# EUROPÄISCHE PATENTSCHRIFT

(11) **EP 3 877 695 B1**
(45) Veröffentlichungstag und Bekanntmachung des Hinweises auf die Patenterteilung: **17.09.2025**
(21) Anmeldenummer: 19802142.0
(22) Anmeldetag: 08.11.2019
(51) Int. Cl.: F21V 15/01, F21V 17/12, F21V 21/116, F21V 21/40, A61B 90/30, F21V 31/00, F21W 131/205, F21Y 105/10, F21Y 115/10

(54) **LEUCHTENGEHÄUSE UND OPERATIONSLEUCHTE MIT LEUCHTENGEHÄUSE**
LIGHT HOUSING AND OPERATION LIGHT WITH LIGHT HOUSING
BOÎTIER D'ÉCLAIRAGE ET LAMPE D'OPÉRATION AVEC UN BOÎTIER D'ÉCLAIRAGE

(30) Priorität: 08.11.2018 DE 202018106344 U
(43) Veröffentlichungstag der Anmeldung: 15.09.2021
(73) Patentinhaber: Karl Leibinger Asset Management GmbH & Co. KG, 78570 Mühlheim an der Donau (DE)
(72) Erfinder: STRÖLIN, Joachim, 78604 Rietheim (DE)
(74) Vertreter: Winter, Brandl - Partnerschaft mbB
(86) Internationale Anmeldenummer: PCT/EP2019/080727
(87) Internationale Veröffentlichungsnummer: WO 2020/094857

(56) Entgegenhaltungen:
- WO-A1-2012/156617
- GB-A- 739 621
- JP-A- 2004 288 474
- US-A- 4 037 096
- US-A1- 2014 268 751

## Beschreibung

Die Erfindung betrifft ein mehrteilig ausgebildetes Leuchtengehäuse für eine medizinische Operationsleuchte, in das zumindest ein Leuchtmittel einsetzbar ist. Das Leuchtengehäuse weist einen Gehäusekörper, ein den Gehäusekörper verschließendes Verschlusselement und eine Lagervorrichtung zum Befestigen des Leuchtengehäuses an einer Tragvorrichtung, wie einem Tragarm, auf. Ferner betrifft die Erfindung eine Operationsleuchte mit einem Leuchtengehäuse, wobei ein Leuchtmittel in das Leuchtengehäuse eingesetzt ist und das Leuchtengehäuse das Leuchtmittel zur Umgebung staubdicht abdichtet.

Aus dem Stand der Technik sind bereits Leuchtengehäuse für Operationsleuchte bekannt. Beispielsweise zeigt die DE 10 2011 102 638 A1 oder die DE 10 2011 102 645 A1 eine mehrteilige Operationsleuchte,. Oftmals sind bei solchen Operationsleuchten ein Oberteil mit einem Unterteil über seitliche Schrauben miteinander verbunden. Zudem ist aus der US 2014/268 751 A1 ein Leuchtengehäuse für eine Operationsleuchte bekannt, die über eine Lagervorrichtung an einem Tragarm gehalten werden kann. Auch ist aus der US 4 037 096 A ein Leuchtengehäuse mit einem Gehäusekörper und einem als Verschlusselement dienenden Handgriff bekannt.

Der Stand der Technik hat jedoch den Nachteil, dass die Operationsleuchten ein Eigengewicht von bis zu 10kg aufweisen und zusätzlich Kräfte zum Bewegen der Operationsleuchte über das Leuchtengehäuse an die Tragvorrichtung weitergegeben werden müssen. Die Stabilität der bisher bekannten Leuchtengehäuse ist nicht ausreichend hoch, um die hohen Kräfte aufnehmen zu können.

Es ist also die Aufgabe der Erfindung, die Nachteile aus dem Stand der Technik zu vermeiden oder wenigstens zu verringern. Insbesondere soll ein Leuchtengehäuse für eine medizinische Operationsleuchte bereitgestellt werden, die besonders stabil, sicher und langlebig ist. Zudem soll der Aufbau des Leuchtengehäuses möglichst einfach und leicht sterilisierbar sein, um einen Einsatz im Operationsraum zu ermöglichen.

Die Aufgabe der Erfindung wird bei einer gattungsgemäßen Vorrichtung erfindungsgemäß durch den Gegenstand des Anspruchs 1 gelöst, wobei das Leuchtengehäuse einen von dem Gehäusekörper separat ausgebildeten Träger besitzt, der als ein Blechbauteil ausgebildet ist, eine Rahmenstruktur bildet und über den eine über das Verschlusselement eingeleitete Kraft, insbesondere zum Bewegen des Leuchtengehäuses bzw. der Operationsleuchte, und/oder eine Gewichtskraft des Leuchtengehäuses an die Lagervorrichtung übertragbar ist. Das heißt also, dass die Kräfte größtenteils nicht von dem Gehäusekörper übertragen werden, sondern allein über die durch den Träger gebildete Tragstruktur von dem Krafteinleitungspunkt, wie einem zentralen Handgriff, zu der Lagervorrichtung und dann an die Tragvorrichtung übertragen wird.

Dies hat den Vorteil, dass ein besonders stabiles Leuchtengehäuse bereitgestellt werden kann. Durch die Verwendung des zusätzlichen Trägers in dem Leuchtengehäuse, über die die aufzunehmenden Kräfte weitergeleitet werden, kann ein besonders sicheres und langlebiges Leuchtengehäuse bereitgestellt werden, da der Gehäusekörper kaum belastet wird. Somit kann ein sicherer Betrieb gewährleistet werden, selbst kein Teile des Leuchtengehäuses, wie der Gehäusekörper, beschädigt werden.

Vorteilhafte Ausführungsformen sind in den Unteransprüchen beansprucht und werden nachfolgend näher erläutert.

Gemäß einer bevorzugten Ausführungsform können der Träger und der Gehäusekörper aus unterschiedlichen Materialien ausgebildet sein. Vorzugsweise weist das Material des Trägers eine höhere Festigkeit und/oder Steifigkeit als das Material des Gehäusekörpers auf. Dadurch, dass die zu übertragenden Kräfte von dem als Tragstruktur dienenden Träger aufgenommen werden, muss der Gehäusekörper keine hohe Festigkeit und Stabilität besitzen. Durch die Reduzierung der Kräfte auf den Gehäusekörper können die Materialanforderungen des Gehäusematerials gesenkt werden.

Besonders bevorzugt ist es, wenn der Träger aus Stahl aufgebaut ist. Dadurch können die Kräfte einfach durch die Struktur aufgenommen werden und weitergegeben werden. Aufgrund seines einfachen Aufbaus und seiner Festigkeit ist eine Stahlträger-Traverse eine besonders bevorzugte Ausführungsform für den Träger.

In einer vorteilhaften Weiterbildung kann das Verschlusselement über ein Verbindungselement, wie beispielsweise Schrauben, vorzugsweise direkt, an dem Träger befestigt sein. Alternativ oder zusätzlich kann das Verschlusselement an dem Gehäusekörper angebracht sein. Durch die Befestigung des Verschlusselements am Träger wird der Kraftfluss vorteilhafterweise nicht über den Gehäusekörper geleitet, sondern direkt zu der Lagervorrichtung des Leuchtengehäuses.

Vorzugsweise ist die Lagervorrichtung direkt an dem Träger befestigt. Beispielsweise kann die Lagervorrichtung durch ein einseitiges Seitenlager gebildet sein.

Gemäß einer bevorzugten Weiterbildung kann das Leuchtengehäuse eine Griffvorrichtung und/oder eine Abdeckvorrichtung aufweisen, wobei die Griffvorrichtung und/oder die Abdeckvorrichtung das Verbindungselement verdeckend an dem Verschlusselement, vorzugsweise austauschbar, befestigbar sind/ist. Das heißt also, dass das Leuchtengehäuse keine äußeren sichtbaren Verbindungselemente besitzt. Dadurch kann das Leuchtengehäuse einfach abgewischt und steril gehalten werden. Da das Verbindungselement ausreichend hohe Kräfte übertragen muss, ist es vorteilhaft, wenn es durch mehrere, beispielsweise vier, Schrauben gebildet ist, die das Verschlusselement mit dem Träger und/oder dem Gehäusekörper verbinden. Somit kann das Leuchtengehäuse einfach über das Verbindungselement geschlossen werden, da nur das Verbindungselement am Verschlusselement nötig ist. Somit ist das Innere des Leuchtengehäuse im Service leicht zugänglich und das Verschlusselement besonders wirtschaftlich befestigt.

Da das Verbindungelement, das üblicherweise aus Metall ausgebildet ist, zusätzlich abgedeckt wird, beispielsweise durch einen Handgriff oder eine andere Abdeckung, kann durch die Wahl eines geeigneten Handgriffs oder einer geeigneten Abdeckung die Sterilisierbarkeit verbessert werden. Ein Handgriff oder eine Abdeckung aus Kunststoff ist besonders vorteilhaft. Zudem ist es bevorzugt, wenn der Handgriff bzw. die Abdeckung auch über eine Verbindungsvorrichtung an dem Verschlusselemente angebracht ist, die besonders einfach abwischbar bzw. sterilisierbar ist. Als geeignete Verbindungsvorrichtung hat/haben sich beispielsweise eine oder mehrere Knebelschrauben erwiesen. Das Vorsehen von verdeckten Verbindungselementen hat sich auch unabhängig von dem Vorsehen des Trägers als vorteilhaft erwiesen.

In einer vorteilhaften Ausführungsform kann der Gehäusekörper ein Oberteil und ein separat von dem Oberteil ausgebildetes, an das Oberteil anschließendes Unterteil besitzen, wobei das Unterteil zumindest im Bereich eines von dem Leuchtmittel hervorgerufenen Strahlengangs lichttransparent ausgebildet ist. Somit kann der Gehäusekörper einfach geöffnet werden, um das Leuchtmittel auszutauschen.

Zudem ist es von Vorteil, wenn das Verschlusselement das Unterteil, vorzugsweise vorgespannt, an dem Oberteil befestigt. Somit kann das Leuchtengehäuse einfach über das Verschlusselement, und damit einfach über das Verbindungselement, geschlossen werden. Zum Schließen des Leuchtengehäuses ist es also nur nötig das Verbindungselement anzubringen, d.h. die Schrauben einzuschrauben. Somit ist das Innere des Leuchtengehäuse im Service leicht zugänglich und das Verschlusselement besonders wirtschaftlich befestigt. Das Oberteil muss somit nur die Schließkraft, mit der das Unterteil an dem Oberteil befestigt wird, aufnehmen. Es wirken also kaum Kräfte auf das Oberteil.

Als vorteilhafte Ausführungsform kann das Oberteil aus Polyurethan aufgebaut sein und/oder das Unterteil aus gehärtetem Glas, wie Mineralglas, aufgebaut sein. Diese Materialien haben sich als besonders geeignet bewährt. Das Unterteil ist vorzugsweise im Wesentlichen flächig ausgestaltet.

Zudem ist es von Vorteil, wenn der Gehäusekörper, insbesondere das Oberteil, an dem Träger angebracht ist. Vorzugsweise ist das Oberteil direkt mit dem Träger verschraubt.

Gemäß einer bevorzugten Weiterbildung kann das Verschlusselement als ein Haltering ausgebildet sein, der zentral an dem Leuchtengehäuse angeordnet ist. Die Kraft wird also zentral über das Verschlusselement eingeleitet und über den Träger an das Seitenlager ausgeleitet. Vorzugsweise ist das Verschlusselement im Wesentlichen achsparallel zu dem Strahlengang des Leuchtmittels angeordnet, so dass das Verschlusselement die Leuchtkraft des Leuchtmittels nicht beeinträchtigt. Beispielsweise kann das Verschlusselement aus Kunststoff ausgebildet sein.

Ferner ist es zweckmäßig, wenn das Verschlusselement einen Ringabschnitt, der eine Aussparung des Unterteils durchgreift, und einen von dem Ringabschnitt radial nach außen abstehenden, vorzugsweise umlaufenden, Flanschabschnitt, der an einer Außenseite des Unterteils anliegt, besitzt. Über den Flanschabschnitt kann die Vorspannkraft auf das Unterteil in Richtung zu dem Oberteil aufgebracht werden. Vorzugsweise ist das Verbindungselement nah am Flanschabschnitt angebracht, so dass Kräfte geeignet übertragen werden können. Durch das Vorsehen des Ringabschnitts kann eine geeignete Anbindungsmöglichkeit für die Griffvorrichtung, wie den Handgriff, gebildet werden. Vorzugsweise liegt (im montierten Zustand) ein dem Flanschabschnitt abgewandtes Ende des Ringabschnitts an einer Innenseite des Oberteils an. Somit kann die Vorspannung aufgebracht werden. Zusätzlich wird dadurch die Stabilität erhöht.

Besonders bevorzugt ist es, wenn das Leuchtengehäuse zum Abdichten eines Inneren des Leuchtengehäuses eine Gehäusekörperdichtung, die zwischen dem Oberteil und dem Unterteil angeordnet ist, und/oder eine Verschlussdichtung , die zwischen dem Unterteil und dem Verschlusselement, insbesondere dem Flanschabschnitt, angeordnet ist, aufweist. Somit kann sicher ausgeschlossen werden, dass zwischen den mehreren Bauteilen des Leuchtengehäuse Staub oder andere Verunreinigungen in das Innere des Leuchtengehäuses eintreten kann.

Die Aufgabe der Erfindung wird auch durch eine Operationsleuchte mit einem solchen Leuchtengehäuse gelöst, wobei ein Leuchtmittel in das Leuchtengehäuse eingesetzt ist und das Leuchtengehäuse das Leuchtmittel zur Umgebung staubdicht abdichtet.

Mit anderen Worten betrifft die Erfindung eine mehrteilige medizinische Operationsleuchte, wobei ein bspw. als Haltering ausgebildetes Verschlussmittel die Operationsleuchte verschließend (in die Operationsleuchte) eingesetzt ist. Im Stand der Technik sind bei Operationsleuchten Verschraubungen sichtbar. So betrifft die Erfindung eine Operationsleuchte / ein Leuchtengehäuse mit einseitiger Lagerung. Sie ist stabil, IP-Dicht (abgedichtet) und ohne sichtbare Verschraubung. Das Leuchtengehäuse wird über das Abschlussglas geschlossen. Die Leuchte hat einen zentralen Handgriff. Hinter diesem abnehmbaren Handgriff sitzt ein zentraler Haltering. Mit diesem Haltering wird das Abschlussglas verschraubt. Das Abschlussglas ist ein gehärtetes Mineralglas, das sehr hohe Kräfte aufnehmen kann. Die zentrale Verschraubung hält zum einen das Glas selbst und zieht es auf die Auflagefläche am Außendurchmesser des Gehäuses. Sowohl der zentrale Befestigungsring als auch die Auflagefläche außen weisen Dichtungen auf. Diese Art, die Leuchte zu schließen, ist sehr einfach. Es sind lediglich vier Schrauben am Haltering notwendig. Sowohl der zentrale Haltering als auch die Schrauben werden vom zentralen Handgriff abgedeckt. Die Krafteinleitung über das Seitenlager erfolgt über einen Stahlträger (Traverse) zum zentralen Haltering. Das Gehäuse selbst ist aus PUR (Polyurethan) und nimmt nur geringe Kräfte auf.

Die Erfindung wird nachfolgend mit Hilfe von Figuren erläutert. Es zeigen:
Fig. 1 eine erste Explosionsdarstellung einer erfindungsgemäßen Operationsleuchte mit einem erfindungsgemäßen Leuchtengehäuse;
Fig. 2 eine zweite Explosionsdarstellung der Operationsleuchte;
Fig. 3 eine Explosionsdarstellung des Leuchtengehäuses; und
Fig. 4 eine perspektivische Darstellung der Operationsleuchte mit einer daran angebrachten Griffvorrichtung.

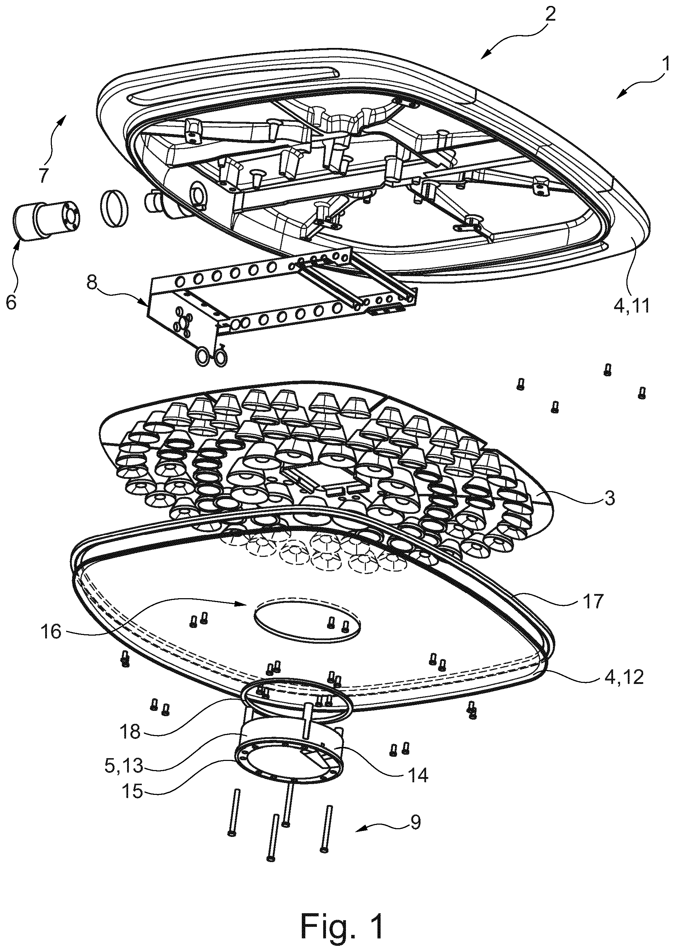

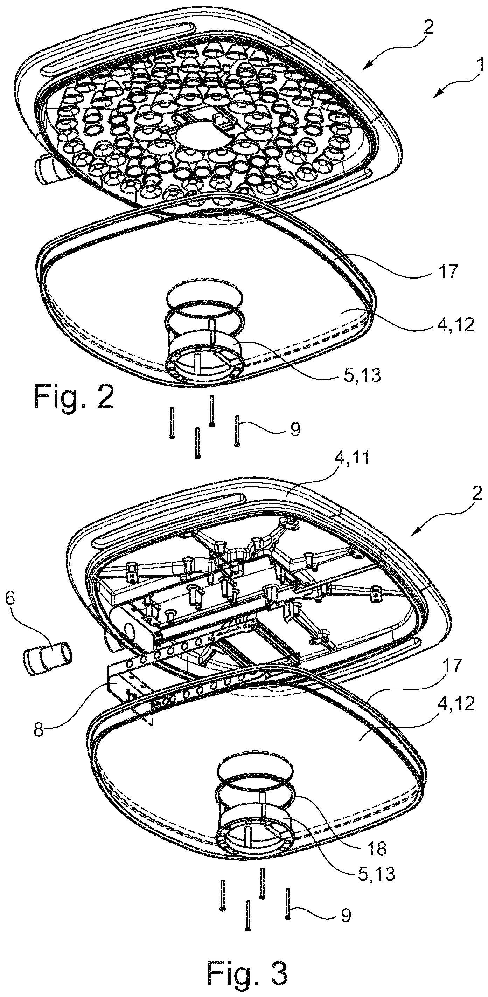

Die Zeichnungen sind leidglich schematischer Natur und dienen dem Verständnis der Erfindung. Die gleichen Elemente sind mit denselben Bezugszeichen gekennzeichnet. Fign. 1 bis 4 zeigen ein erfindungsgemäßes Leuchtengehäuse 1 einer Operationsleuchte 2. In dem Leuchtengehäuse 1 ist zumindest ein Leuchtmittel 3 zum Umgebung staubdicht abgedichtet einsetzbar. Das Leuchtmittel 3 ist in den dargestellten Ausführungsformen durch ein Leuchtenfeld gebildet.

Das Leuchtengehäuse 1 weist einen Gehäusekörper 4, ein den Gehäusekörper 4 verschließendes Verschlusselement 5 und eine Lagervorrichtung 6 zum Befestigen des Leuchtengehäuses 1 an einer Tragvorrichtung 7 auf. Die Tragvorrichtung 7 wird üblicherweise durch einen Tragarm gebildet, an dem die Operationsleuchte 2 angebracht ist. Die Tragvorrichtung 7 ist in den Zeichnungen nicht explizit dargestellt.

Erfindungsgemäß besitzt das Leuchtengehäuse 1 einen Träger 8. über den eine über das Verschlusselement 5 eingeleitete Kraft, insbesondere zum Verlagern der Operationsleuchte 2, und/oder eine Gewichtskraft des Leuchtengehäuses 1 an die Lagervorrichtung 6 übertragbar ist. Der Träger 8 ist separat von dem Gehäusekörper 4 ausgebildet. Der Träger 8 bildet eine Tragstruktur für die Operationsleuchte 2. Der Träger 8 und der Gehäusekörper 4 sind aus unterschiedlichen Materialien ausgebildet. In den dargestellten Ausführungsformen ist der Träger 8 durch eine Stahlträger-Traverse gebildet. Der Träger 8 ist als ein Blechbauteil ausgebildet, der eine Rahmenstruktur bildet. Der Träger 8 ist im Inneren des Leuchtengehäuses 1 angeordnet.

Der Träger 8 ist direkt mit der Lagervorrichtung 6, durch den Gehäusekörper 4 hindurch, verbunden. Die Lagervorrichtung 6 ist als ein einseitiges Seitenlager ausgebildet. Das Verschlusselement 5 ist über ein Verbindungselement 9 an dem Träger 8 befestigt. In der dargestellten Ausführungsform wird das Verbindungselement 9 durch eine Vielzahl an Schrauben, hier vier Schrauben gebildet. Die Schrauben werden in einer Richtung entlang des Strahlengangs des Leuchtmittels 3 eingeschraubt. Das Verschlusselement ist also direkt über das Verbindungselement 9 an dem Träger 8 befestigt. Somit wird die Kraft direkt, d.h. nicht über den Gehäusekörper 4, an den Träger 8 weitergegeben. Der Gehäusekörper 4 ist auch an dem Träger 8 befestigt. Der Gehäusekörper 4 überträgt im Wesentlichen keine Kräfte, die durch das Bewegen der Operationsleuchte 2 und/oder das Gewicht der Operationsleuchte 2 entstehen.

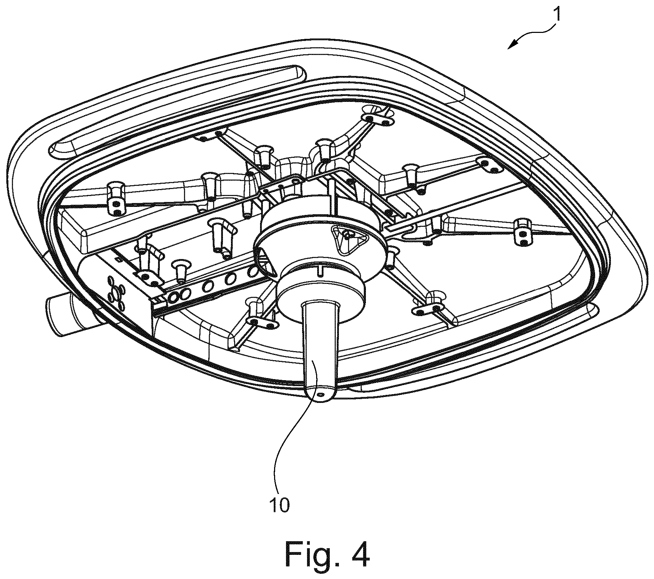

Das Verbindungselement 9 ist nach außen hin abgedeckt. Das heißt, es gibt keine äußerlichen Schrauben. In der dargestellten Ausführungsform (vgl. Fig. 4) weist das Leuchtengehäuse 1 eine Griffvorrichtung 10 auf, die das Verbindungselement 9 verdeckend an dem Verschlusselement 5 befestigt ist. Die Griffvorrichtung 10 ist als ein Handgriff ausgebildet. Die Griffvorrichtung 10 ist zentral/mittig an einer Außenseite des Leuchtengehäuses bzw. des Gehäusekörpers 4 angebracht. Die Griffvorrichtung 10 kann ausgetauscht werden. Die Griffvorrichtung 10 ist in der dargestellten Ausführungsform über eine oder mehrere Schrauben, hier Knebelschrauben, an dem Verschlusselement 5 angebracht. Alternativ oder zusätzlich kann das Leuchtengehäuse 1 zum Abdecken des Verbindungselements 9 eine Abdeckvorrichtung aufweisen, die das Verbindungselement 9 verdeckend an dem Verschlusselement 5 befestigt ist.

Der Gehäusekörper 4 ist mehrteilig aufgebaut. Der Gehäusekörper 4 weist ein Oberteil 11 und ein separat von dem Oberteil 11 ausgebildetes Unterteil 12 auf. Das Unterteil 12 schließt an das Oberteil 11 an. Das Unterteil 12 ist als ein Abschlussglas ausgebildet. Das Unterteil 12 ist zumindest im Bereich eines von dem Leuchtmittel 3 hervorgerufenen Strahlengangs lichttransparent ausgebildet. Das Unterteil 12 ist durch das Verschlusselement 5 an dem Oberteil 11 befestigt. Das Verschlusselement 5 spannt das Unterteil 12 gegen das Oberteil 11 vor. Dadurch wird das Unterteil 12 gegen das Oberteil 11 gedrückt, so dass eine dichte Verbindung entsteht. Vorzugsweise ist das Oberteil 11 aus Polyurethan aufgebaut. Vorzugsweise ist das Unterteil 12 aus gehärtetem Glas, beispielsweise aus Mineralglas, aufgebaut.

Das Verschlusselement 5 ist als ein Haltering 13 ausgebildet. Das Verschlusselement 5 ist zentral an dem Leuchtengehäuse 1 angeordnet. Das Verschlusselement 5 weist einen Ringabschnitt 14 und einen Flanschabschnitt 15 auf. Der Ringabschnitt durchgreift eine Aussparung 16 des Unterteils 12. Der Flanschabschnitt 15 steht radial nach außen von dem Ringabschnitt 14 ab. Im montierten Zustand liegt ein dem Flanschabschnitt 15 abgewandtes Ende an dem Oberteil 11 an. Der Flanschabschnitt 15 liegt an einer Außenseite des Unterteils 12 an. Dadurch drückt der Flanschabschnitt 15 das Unterteil 11 gegen das Oberteil. Das Verbindungselement 9 durchgreift das Verschlusselement 5 radial innerhalb des Flanschabschnitts 15.

Das Leuchtengehäuse 1 weist zum Abdichten eines Inneren des Leuchtengehäuses 1 eine Gehäusekörperdichtung 17 auf. Die Gehäusekörperdichtung 17, ist zwischen dem Oberteil 11 und dem Unterteil 12 angeordnet. Das Leuchtengehäuse 1 weist zum Abdichten eines Inneren des Leuchtengehäuses 1 eine Verschlussdichtung 18 auf. Die Verschlussdichtung 18 ist zwischen dem Unterteil 12 und dem Verschlusselement 5 angeordnet. Insbesondere ist die Verschlussdichtung 18 zwischen dem Flanschabschnitt 15 und einer Außenseite des Unterteils 12 angeordnet. Vorzugsweise schließt die Verschlussdichtung 18 radial außerhalb der Aussparung 16 diese Aussparung 16 umschließend an.

## Patentansprüche

1. Leuchtengehäuse (1) für eine Operationsleuchte (2), in das zumindest ein Leuchtmittel (3) einsetzbar ist, mit einem Gehäusekörper (4), einem den Gehäusekörper (4) verschließenden Verschlusselement (5) und einer Lagervorrichtung (6) zum Befestigen des Leuchtengehäuses (1) an einer Tragvorrichtung (7), **dadurch gekennzeichnet, dass** das Leuchtengehäuse (1) einen von dem Gehäusekörper (4) separat und als Blechbauteil ausgebildeten Träger (8) besitzt, der ferner eine Rahmenstruktur bildet und über den eine über das Verschlusselement (5) eingeleitete Kraft und/oder eine Gewichtskraft des Leuchtengehäuses (1) an die Lagervorrichtung (6) übertragbar ist.

2. Leuchtengehäuse (1) nach Anspruch 1, **dadurch gekennzeichnet, dass** das Verschlusselement (5) über ein Verbindungselement (9) an dem Träger (8) befestigt ist.

3. Leuchtengehäuse (1) nach Anspruch 2, **dadurch gekennzeichnet, dass** das Leuchtengehäuse (1) eine Griffvorrichtung (10) und/oder eine Abdeckvorrichtung aufweist, wobei die Griffvorrichtung (10) und/oder die Abdeckvorrichtung das Verbindungselement (9) verdeckend an dem Verschlusselement (5) befestigbar ist.

4. Leuchtengehäuse (1) nach einem der Ansprüche 1 bis 3, **dadurch gekennzeichnet, dass** der Gehäusekörper (4) ein Oberteil (11) und ein separat von dem Oberteil (11) ausgebildetes, an das Oberteil (11) anschließendes Unterteil (12) besitzt, wobei das Unterteil (12) zumindest im Bereich eines von dem Leuchtmittel (3) hervorgerufenen Strahlengangs lichttransparent ausgebildet ist.

5. Leuchtengehäuse (1) nach Anspruch 4, **dadurch gekennzeichnet, dass** das Verschlusselement (5) das Unterteil (12) an dem Oberteil (11) befestigt.

6. Leuchtengehäuse (1) nach Anspruch 4 oder 5, **dadurch gekennzeichnet, dass** der Träger (8) aus Stahl aufgebaut ist und/oder das Oberteil (11) aus Polyurethan aufgebaut ist und/oder das Unterteil (12) aus gehärtetem Glas aufgebaut ist.

7. Leuchtengehäuse (1) nach einem der Ansprüche 1 bis 6, **dadurch gekennzeichnet, dass** das Verschlusselement (5) als ein Haltering (13) ausgebildet ist, der zentral an dem Leuchtengehäuse (1) angeordnet ist.

8. Leuchtengehäuse (1) nach einem der Ansprüche 4 bis 7, **dadurch gekennzeichnet, dass** das Verschlusselement (5) einen Ringabschnitt (14), der eine Aussparung (16) des Unterteils (12) durchgreift, und einen von dem Ringabschnitt (14) radial nach außen abstehenden Flanschabschnitt (15), der an einer Außenseite des Unterteils (12) anliegt, besitzt.

9. Leuchtengehäuse (1) nach einem der Ansprüche 4 bis 8, **dadurch gekennzeichnet, dass** das Leuchtengehäuse (1) zum Abdichten eines Inneren des Leuchtengehäuses (1) eine Gehäusekörperdichtung (17), die zwischen dem Oberteil (11) und dem Unterteil (12) angeordnet ist, und/oder eine Verschlussdichtung (18), die zwischen dem Unterteil (12) und dem Verschlusselement (5) angeordnet ist, aufweist.

10. Leuchtengehäuse (1) nach einem der Ansprüche 1 bis 9, **dadurch gekennzeichnet, dass** der Träger (8) und der Gehäusekörper (4) aus unterschiedlichen Materialien ausgebildet sind, wobei das Material des Trägers (8) eine höhere Festigkeit und/oder Steifigkeit als das Material des Gehäusekörpers (4) aufweist.

11. Operationsleuchte (2) mit einem Leuchtengehäuse (1) nach einem der Ansprüche 1 bis 10, wobei ein Leuchtmittel (3) in das Leuchtengehäuse (1) eingesetzt ist und das Leuchtengehäuse (1) das Leuchtmittel (3) zur Umgebung staubdicht abdichtet.

## Claims

1. A lamp housing (1) for an operating lamp (2), wherein at least on illuminant (3) can be installed in the lamp housing (1), which comprises a housing body (4), a closure element (5) which closes the housing body (4), and a bearing device (6) for fastening the lamp housing (1) to a supporting device (7), **characterized in that** the lamp housing (1) has a support (8), which is formed separately from the housing body (4) and as a sheet metal component, which furthermore forms a frame structure, and wherein by means of the support (8), a force introduced via the closure element (5) and/or a weight force of the lamp housing (1) can be transferred to the bearing device (6).

2. The lamp housing (1) according to claim 1, **characterized in that** the closure element (5) is fastened to the support (8) via a connection element (9).

3. The lamp housing (1) according to claim 2, **characterized in that** the lamp housing (1) includes a gripping device (10) and/or a covering device, wherein the gripping device (10) and/or the covering device can be fastened to the closure element (5) while covering the connection element (9).

4. The lamp housing (1) according to one of the claims 1 to 3, **characterized in that** the housing body (4) has an upper part (11) and a lower part (12) formed separately from the upper part (11) and being connected to the upper part (11), the lower part (12) being light-transparent at least in the region of an optical path produced by the illuminant (3).

5. The lamp housing (1) according to claim 4, **characterized in that** the closure element (5) fastens the lower part (12) to the upper part (11).

6. The lamp housing (1) according to claim 4 or 5, **characterized in that** the support (8) is made from steel and/or the upper part (11) is made from polyurethane and/or the lower part (12) is made from tempered glass.

7. The lamp housing (1) according to one of the claims 1 to 6, **characterized in that** the closure element (5) is in the form of a holding ring (13) which is disposed centrally on the lamp housing (1).

8. The lamp housing (1) according to one of the claims 4 to 7, **characterized in that** the closure element (5) includes an annular portion (14) which passes through a recess (16) of the lower part (12) and a flange portion (15) projecting radially outward from the annular portion (14), wherein theflange portion (15) abuts on an outer face of the lower part (12).

9. The lamp housing (1) according to one of the claims 4 to 8, **characterized in that**, for sealing an interior of the lamp housing (1), the lamp housing (1) includes a housing body seal (17) interposed between the upper part (11) and the lower part (12) and/or a closure seal (18) interposed between the lower part (12) and the closure element (5).

10. The lamp housing (1) according to one of claims 1 to 9, **characterized in that** the carrier (8) and the housing body (4) are made of different materials, wherein the material of the carrier (8) has a higher strength and/or rigidity than the material of the housing body (4).

11. An operating lamp (2) comprising a lamp housing (1) according to one of the claims 1 to 10, wherein an illuminant (3) is installed in the lamp housing (1) and the lamp housing (1) seals the illuminant (3) against the environment in a dust-tight manner.

## Revendications

1. Boîtier d'éclairage (1) pour une lampe scialytique (2), dans lequel au moins un moyen d'éclairage (3) peut être inséré, avec un corps de boîtier (4), un élément de fermeture (5) fermant le corps de boîtier (4) et un dispositif de palier (6) pour la fixation du boîtier d'éclairage (1) à un dispositif porteur (7), **caractérisé en ce que** le boîtier d'éclairage (1) possède un support (8) formé comme composant de tôle et séparé du corps de boîtier (4), support qui forme de plus une structure de cadre et par le biais duquel une force introduite par le biais de l'élément de fermeture (5) et/ou une force pondérale du boîtier d'éclairage (1) peut être transmise au dispositif de palier (6).

2. Boîtier d'éclairage (1) selon la revendication 1, **caractérisé en ce que** l'élément de fermeture (5) est fixé au support (8) par le biais d'un élément de liaison (9).

3. Boîtier de luminaire (1) selon la revendication 2, **caractérisé en ce que** le boîtier de luminaire (1) présente un dispositif de préhension (10) et/ou un dispositif de recouvrement, dans lequel le dispositif de préhension (10) et/ou le dispositif de recouvrement peut être fixé à l'élément de fermeture (5) en recouvrant l'élément de liaison (9).

4. Boîtier d'éclairage (1) selon l'une quelconque des revendications 1 à 3, **caractérisé en ce que** le corps de boîtier (4) possède une partie supérieure (11) et une partie inférieure (12) formée séparément de la partie supérieure (11), raccordée à la partie supérieure (11), dans lequel la partie inférieure (12) est formée de manière transparente à la lumière au moins dans la zone d'un trajet de faisceau suscité par le moyen d'éclairage (3).

5. Boîtier d'éclairage (1) selon la revendication 4, **caractérisé en ce que** l'élément de fermeture (5) fixe la partie inférieure (12) à la partie supérieure (11).

6. Boîtier d'éclairage (1) selon la revendication 4 ou 5, **caractérisé en ce que** le support (8) est constitué d'acier et/ou la partie supérieure (11) est constituée de polyuréthane et/ou la partie inférieure (12) est constituée de verre durci.

7. Boîtier d'éclairage (1) selon l'une quelconque des revendications 1 à 6, **caractérisé en ce que** l'élément de fermeture (5) est formé comme un anneau de retenue (13) qui est agencé de manière centrale au niveau du boîtier d'éclairage (1).

8. Boîtier d'éclairage (1) selon l'une quelconque des revendications 4 à 7, **caractérisé en ce que** l'élément de fermeture (5) possède une section annulaire (14) qui traverse un évidement (16) de la partie inférieure (12), et une section de bride (15) de la section annulaire (14) dépassant radialement vers l'extérieur, section de bride qui repose contre un côté extérieur de la partie inférieure (12).

9. Boîtier d'éclairage (1) selon l'une quelconque des revendications 4 à 8, **caractérisé en ce que** le boîtier d'éclairage (1) présente pour étanchéifier un intérieur du boîtier d'éclairage (1) un joint d'étanchéité de corps de boîtier (17) qui est agencé entre la partie supérieure (11) et la partie inférieure (12), et/ou un joint de fermeture (18) qui est agencé entre la partie inférieure (12) et l'élément de fermeture (5).

10. Boîtier d'éclairage (1) selon l'une quelconque des revendications 1 à 9, **caractérisé en ce que** le support (8) et le corps de boîtier (4) sont formés à partir de matériaux différents, dans lequel le matériau du support (8) présente une résistance et/ou une rigidité supérieure à celle du matériau du corps de boîtier (4).

11. Lampe scialytique (2) avec un boîtier d'éclairage (1) selon l'une quelconque des revendications 1 à 10, dans laquelle un moyen d'éclairage (3) est inséré dans le boîtier d'éclairage (1), et le boîtier d'éclairage (1) rend étanche à la poussière le moyen d'éclairage (3) par rapport à l'environnement.
